# EUROPEAN PATENT SPECIFICATION

(11) **EP 4 262 728 B1**
(45) Date of publication and mention of the grant of the patent: **03.07.2024**
(21) Application number: 21819511.3
(22) Date of filing: 06.12.2021
(51) Int. Cl.: A61K 31/4412, A61P 17/00

(54) **ANTI-DANDRUFF COMPOSITION COMPRISING PIROCTONE OLAMINE**
ANTI-SCHUPPEN ZUSAMMENSETZUNG ENTHALTEND PIROCTONE OLAMINE
COMPOSITION ANTI-PELLICULAIRE COMRENANT DE LA PIROCTONE OLAMINE

(30) Priority: 18.12.2020 WO PCT/CN2020/137673; 26.01.2021 EP 21153532
(43) Date of publication of application: 25.10.2023
(73) Proprietor: Unilever IP Holdings B.V., 3013 AL Rotterdam (NL); Unilever Global IP Limited, Wirral, Merseyside CH62 4ZD (GB)
(72) Inventor: CAO, Feng, Shanghai 200335 (CN); CHEN, Guoqiang, Shanghai 200335 (CN); JI, Chengdong, Shanghai 200335 (CN)
(74) Representative: Mathai, Neenu Grace
(86) International application number: PCT/EP2021/084303
(87) International publication number: WO 2022/128551

(56) References cited:
- TR-A2- 202 012 039
- Anonymous: "Shampooing antipelliculaire piroctone olamine Casino, le bon choix d'actif | Regard sur les cosmétiques", , 7 October 2020 (2020-10-07), XP055822036, Retrieved from the Internet: URL:https://www.regard-sur-les-cosmetiques .fr/nos-regards/shampooing-antipelliculair e-piroctone-olamine-casino-le-bon-choix-d- actif-1599/ [retrieved on 2021-07-07]
- Olamine Piroctone: "Piroctone olamine", , 23 September 2009 (2009-09-23), XP055822040, Retrieved from the Internet: URL:https://purelyprofessional.dk/wp-conte nt/uploads/inci/piroctone-olamine.pdf [retrieved on 2021-07-07]
- DOROTA KO?ODY?SKA ET AL: "Application of a New-Generation Complexing Agent in Removal of Heavy Metal Ions from Aqueous Solutions", INDUSTRIAL & ENGINEERING CHEMISTRY RESEARCH, vol. 47, no. 9, 1 May 2008 (2008-05-01), pages 3192-3199, XP055384041, ISSN: 0888-5885, DOI: 10.1021/ie701742a
- Hao Xiang ET AL: "Synthesis, crystal structure and magnetic property of a binuclear iron(III) citrate complex", Transition metal chemistry (Weinheim), 1 August 2001 (2001-08-01), pages 384-387, XP055822141, Dordrecht DOI: 10.1023/A:1011055306645 Retrieved from the Internet: URL:https://link.springer.com/content/pdf/ 10.1023/A:1011055306645.pdf [retrieved on 2021-07-07]

## Description

### Field of the Invention

The present invention relates to hair care compositions. More particularly the invention relates to hair care compositions that comprise piroctone olamine.

### Background of the Invention

Dandruff is a condition experienced by many people worldwide. The dandruff condition varies from mild symptoms such as flaking skin to severe inflammation and itchiness of the scalp. Malassezia yeasts, such as *Malassezia furfur,* are believed by some to be the main cause of dandruff and, whilst this might not represent the full scientific picture of the situation, Malassezia yeasts do appear to be closely associated with dandruff. Hence, the strategy conventionally used for the treatment of dandruff is the topical application of antifungals such as zinc pyrithione (ZnPTO), piroctone olamine, climbazole and ketoconazole which are normally delivered through a shampoo. Piroctone olamine (also known as Octopirox^{®}) is a compound often used in the treatment of fungal infections. Piroctone olamine is the ethanolamine salt of the hydroxamic acid derivative piroctone. Piroctone olamine has anti-fungal properties that make it ideal for controlling the root cause of dandruff, a commonly occurring fungus called Malassezia globosa. It is often used in anti-dandruff shampoo as a replacement for the commonly used compound zinc pyrithione. Generally, piroctone olamine is dissolved in the base composition of a shampoo which includes surfactants, water, silicones, polymers and colouring and fragrance ingredients. When a user applies the compositions to hair and scalp, some compound of piroctone olamine get deposited thereon.

Generally, efficacy of an antidandruff shampoo or conditioner depends on the amount of antidandruff active deposited on the scalp and hair because more deposition means more bioavailability. Such shampoos are known: e.g. https://www.regard-sur-les-cosmetiques.fr/nos-regards/shampooing-antipelliculaire-piroctone-olamine-casino-le-bon-choix-d-actif-1599/.

However, the presence of metal salts can reduce the efficacy of piroctone olamine because the deposited piroctone olamine is less bioavailable as piroctone olamine tends to react with metal salts. It is known that the metal salts can be introduced by raw materials and manufacturing, tap water during and after applying the products, and sweat in daily life. Therefore, there is a need to develop products that mitigate this phenomenon and provide higher efficacy.

### Summary of the Invention

The present inventors have determined a new way of improving the bioactivity of antidandruff agent such as piroctone olamine. We have determined that stability of piroctone olamine can be improved if piroctone olamine, a polyaspartate or its derivative and a specific surfactant system are co-formulated into a composition. Besides, it is also believed by the inventors that the polyaspartate or its derivative can improve the bioactivity of piroctone olamine after the piroctone olamine is deposited on the scalp because the polyaspartate or its derivative can be easily deposited on the scalp upon application.

In accordance with a first aspect, disclosed is a hair care composition comprising:
(i) an anionic surfactant;
(ii) an amphoteric or zwitterionic surfactant;
(iii) piroctone olamine;
(iv) a polyaspartate or its derivative presented by the structure of Formula 1 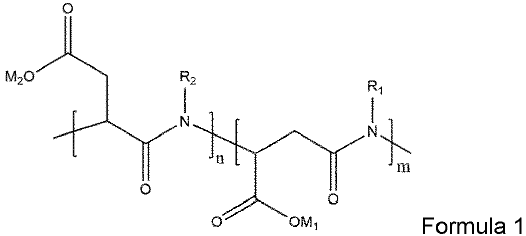 where the sum of m+n is from 2 to 10000, R1, R2 are independently selected from the group consisting of H, OH, (O)ₘG, m is 0 or 1, G is independently selected from the group consisting of H and a substituted or unsubstituted C1-C12 organic group, M1, M2 are independently selected from the group consisting of H, metal salt or ammonium salt, wherein non-limiting examples of the metal salts include Na, K, Ca, Mg, Al salts; and
(v) a cosmetically acceptable carrier.
wherein the weight ratio of said polyaspartate or its derivative to said piroctone olamine in the composition is at least 2:3.

In accordance with a second aspect, disclosed is said composition for use in a method of treating dandruff comprising a step of topical applying the composition of the first aspect on to a desired scalp surface.

### Detailed Description of the Invention

For the avoidance of doubt, any feature of one aspect of the present invention may be utilized in any other aspect of the invention. The word "comprising" is intended to mean "including" but not necessarily "consisting of or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. As used herein, the indefinite article "a" or "an" and its corresponding definite article "the" means at least one, or one or more, unless specified otherwise. The various features of the present invention referred to in individual sections above apply, as appropriate, to other sections *mutatis mutandis.* Consequently, features specified in one section may be combined with features specified in other sections as appropriate. Any section headings are added for convenience only, and are not intended to limit the disclosure in any way.

By "hair care composition" as used herein, is meant to include a composition for topical application to hair or scalp of mammals, especially humans. By topical is meant that the composition is applied to the external surface of the body. In the present invention this is achieved by applying the composition on the hair or scalp. Such a composition may be generally classified as leave-on or rinse off, and includes any product applied for improving the appearance, cleansing, odor control or general aesthetics of scalp and hair. The haircare composition of the present invention could be in the form of a liquid, lotion, cream, foam, scrub, gel, shampoo, conditioner, shower gel or bar. The haircare composition of the present invention is preferably a leave-on composition. Alternatively, the haircare composition of the present invention is a wash-off composition. Compositions for achieving the desired benefits by way of ingestion into the human body are excluded from the scope of the present invention.

### Polyaspartate or its derivative

The composition in accordance with this invention comprises a polyaspartate or its derivative represented by the structure of Formula 1: 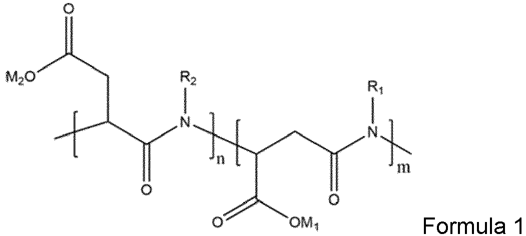 where the sum of m+n is from 2 to 10000, R1, R2 are independently selected from the group consisting of H, OH, (O)ₘG, m is 0 or 1, G is independently selected from the group consisting of H and a substituted or unsubstituted C1-C12 organic group, M1, M2 are independently selected from the group consisting of H, metal salt or ammonium salt, wherein non-limiting examples of the metal salts include Na, K, Ca, Mg, Al salt.

It is preferred that when m is 0, G is independently selected from the group consisting a substituted or unsubstituted C1-C9 organic group, when m is 1, G is independently selected from the group consisting a substituted or unsubstituted C1-C2 organic group.

It is preferred that the polyaspartate or its derivative can be represented by the structure of formula 1 where the sum of m+n is from 2 to 10000, R1, R2 are independently selected from the group consisting of H, OH, M1, M2 are independently selected from the group consisting of H, metal salt or ammonium salt, wherein non-limiting examples of the metal salts include Na, K, Ca, Mg, Al salts.

It is preferred that the polyaspartate or its derivative can be represented by the structure of formula 1 where the sum of m+n is from 2 to 10000, R1, R2 are H, M1, M2 are independently selected from the group consisting of H, metal salt or ammonium salt, wherein non-limiting examples of the metal salts include Na, K, Ca, Mg, Al salts.

It is more preferred that the polyaspartate or its derivative can be represented by the structure of formula 1 the sum of m+n is from 10 to 50, R1, R2 are H, M1, M2 are independently selected from the group consisting of H, metal salt or ammonium salt, wherein non-limiting examples of the metal salts include Na, K, Ca, Mg, Al salts.

It is particularly preferred that the polyaspartate derivative is sodium polyaspartate.

The amount of polyaspartate or its derivative in the composition of the invention would depend on the amount of piroctone olamine used. It is preferred that the composition comprises 0.1 to 10 wt% of polyaspartate or its derivative, more preferably 0.5 to 5 wt%, furthermore preferably 1 to 2 wt% by weight of the composition.

Without wishing to be bound by theory, the present inventors believe that the polymer or polymer derivatives being able to complex with metal ions can improve the stability of piroctone olamine during the application of the composition but also improve the bioactivity of piroctone olamine after being deposed on the scalp because it's easier to deposit polymer or polymer derivatives on scalp comparing with other common metal chelators (for example, EDTA, citric acid, etc.).

Surprisingly, it was found by the inventors that the polyaspartate or its derivative has the better performance regarding improving the stability of piroctone olamine comparing with other polymer or polymer derivatives.

Besides, it is also believed by the inventors that the can improve the bioactivity of piroctone olamine after the piroctone olamine is deposited on the scalp because the polyaspartate or its derivative can be easily deposited on the scalp upon application.

### Piroctone olamine

Piroctone Olamine is an olamine salt of the hydroxamic acid derivative piroctone which is a typical antimicrobial active. It is commonly known as piroctone ethanolamine with the trade name Octopirox^{®}.

The piroctone olamine according to the present invention is a 1:1 compound of 1-hydroxy-4-methyl-6-(2,4,4-trimethylpentyl)-2(1*H*)-pyridinone with 2-aminoethanol and is also designated 1-hydroxy-4-methyl-6-(2,4,4-trimethylpentyl)-2(7*H*) pyridinone monoethanolamine salt. The CAS number is 68890-66-4 and the compound has the general formula as below:

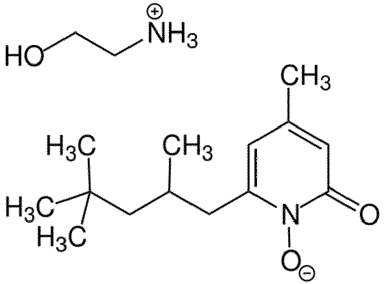

Several antidandruff hair care products contain piroctone olamine. It is found that dissolved intact piroctone olamine molecules are the sole bioactive form of piroctone olamine. When a hair care product with piroctone olamine is utilized, the piroctone olamine dissociates and forms complex with the metal ions (e.g. iron ions, etc) sourced from tap water or sweat due to which stability of the bioactive is affected which reduces its antidandruff efficacy.

Amount of the piroctone olamine in the composition of the invention would depend on the type of the hair care composition and the precise nature of other antidandruff agents used. It is preferred that the composition comprises 0.1 to 10 wt% of said piroctone olamine, more preferably 0.5 to 5 wt%, furthermore preferably 0.5 to 2 wt% by weight of the composition.

The weight ratio of the polyaspartate or its derivatives to the piroctone olamine in the composition is at least 2:3, preferably at least 1:1, more preferably at least 2:1. It is preferably the weight ratio of the polyaspartate or its derivatives to the piroctone olamine in the composition is from 2:3 to 85:1, preferably from 1:1 to 85:1, more preferably from 2:3 to 20:1, more preferably 1:1 to 10:1, furthermore preferably from 2:1 to 10:1, most preferably 2:1 to 5:1.

### Surfactant

The composition in accordance with this invention comprises an anionic surfactant.

Non-limiting examples of suitable anionic cleansing surfactants are alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule. Typical anionic cleansing surfactants for use in compositions of the invention include, but not limited to, sodium oleyl succinate, ammonium lauryl sulphosuccinate, sodium lauryl sulphate, sodium lauryl ether sulphate, sodium lauryl ether sulphosuccinate, ammonium lauryl sulphate, sodium dodecylbenzene sulphonate, triethanolamine dodecylbenzene sulphonate, sodium cocoyl isethionate, sodium lauryl isethionate, lauryl ether carboxylic acid, sodium N-lauryl sarcosinate or mixtures thereof.

Preferred anionic surfactants are the alkyl sulphates and alkyl ether sulphates. Preferred alkyl sulphates are C8-18 alky sulphates, more preferably C12-18 alkyl sulphates, preferably in the form of a salt with a solubilising cation such as sodium, potassium, ammonium or substituted ammonium. Examples are sodium lauryl sulphate (SLS) or sodium dodecyl sulphate (SDS). It is particularly preferred that the anionic cleansing surfactant is alkyl ether sulphate. Preferred alkyl ether sulphates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulphate (SLES). Preferred alkyl ether sulphate is sodium lauryl ether sulphate having an average degree of ethoxylation of from 0.5 to 3, preferably from 1 to 3, more preferably from 2 to 3.

These anionic surfactants are preferably present at a level of from 2 to 16%, more preferably from 3 to 16% by weight of the composition

The composition in accordance with this invention comprises an amphoteric or zwitterionic surfactant. Suitable examples include alkyl amine oxides, alkyl betaines, alkyl amidopropyl betaines, alkyl sulphobetaines (sultaines), alkyl amphoacetates, alkyl amphopropionates, alkyl amidopropyl hydroxysultaines, wherein the alkyl group has from 8 to 19 carbon atoms.

Preferably, the amphoteric surfactant is a betaine surfactant. It is preferred that the betaine surfactant is one of alkyl amidopropyl betaines. Cocamidopropyl betaine (CAPB) is particularly preferred.

In a preferred embodiment, the composition comprises from 0.1 to 10 wt.%, preferably from 0.5 to 8 wt.%, more preferably from 1 to 5 wt.% of an amphoteric surfactant.

### Hair care composition

In accordance with a further aspect of the invention is disclosed a hair care composition of the first aspect. Preferably the composition is a shampoo composition.

Preferably, the hair care composition of the present invention comprises from 1 to 50%, preferably from 2 to 40%, more preferably from 4 to 25% total surfactant.

Suitably, the shampoo composition comprises 50 to 98%, preferably from 60 to 92% water.

Preferably the shampoo composition comprises one or more cationic polymers for conditioning the hair.

Suitable cationic polymers include homopolymers which are cationically substituted or may be formed from two or more types of monomers. The weight average (M_{w}) molecular weight of the polymers will generally be between 100000 and 3 Million Daltons.

The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus, when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/g. The cationic charge density of the polymer is suitably determined via the Kjeldahl method as described in the US Pharmacopoeia under chemical tests for nitrogen determination.

Suitable cationic polymers include copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general, secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable (non-limiting examples of) cationic polymers include:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides (as described in WO95/22311).

Other cationic polymers that can be used include cationic polysaccharide polymers, such as cationic cellulose derivatives, cationic starch derivatives, and cationic guar gum derivatives.

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14 and JAGUAR C17.

Mixtures of any of the above cationic polymers may be used.

It is preferred that the hair care composition of the invention comprises 0.01 to 5%, preferably from 0.02 to 1%, more preferably from 0.05 to 0.8% cationic polymer.

The hair care composition of the invention may additionally comprise a cationic deposition polymer which is a cationic polygalactomannans having an average molecular weight (*M_{w}*) of from 1 million to 2.2 million g/mol and a cationic degree of substitution of from 0.13 to 0.3.

The polygalactomannans are polysaccharides composed principally of galactose and mannose units and are usually found in the endosperm material of seeds from leguminous plants such as guar, locust bean, honey locust, flame tree, and other members of the *Leguminosae* family. Polygalactomannans are composed of a backbone of 1→4-linked β-D-mannopyranosyl main chain units (also termed mannoside units or residues) with recurring 1→6-linked α-D-galactosyl side groups (also termed galactoside units or residues) branching from the number 6 carbon atom of a mannopyranose residue in the polymer backbone. The polygalactomannans of the different *Leguminosae* species differ from one another in the frequency of the occurrence of the galactoside side units branching from the polymannoside backbone. The mannoside and galactoside units are generically referred to herein as glycoside units or residues. The average ratio of mannoside to galactoside units in the polygalactomannan contained in guar gum (hereinafter termed "guar") is approximately 2:1.

Suitable cationic polygalactomannans include guar and hydroxyalkyl guar (for example hydroxyethyl guar or hydroxypropyl guar), that has been cationically modified by chemical reaction with one or more derivatizing agents.

In a typical composition the amount of cationic polygalactomannans will generally range from about 0.05 to 1%, preferably from 0.1 to 0.8%, more preferably 0.2 to 0.6% by weight of the composition.

The hair care composition of the invention may additionally comprise an anionic polymeric rheology modifier such as a carboxylic acid polymer.

The term "carboxylic acid polymer" in the context of this invention generally denotes a homopolymer or copolymer obtained from the polymerization of ethylenically unsaturated monomers containing pendant carboxylic acid groups (hereinafter termed "carboxylic monomers").

Suitable carboxylic monomers generally have one or two carboxylic acid groups, one carbon to carbon double bond and contain a total of from 3 to about 10 carbon atoms, more preferably from 3 to about 5 carbon atoms.

Specific examples of suitable carboxylic monomers include α-β-unsaturated monocarboxylic acids such as acrylic acid, methacrylic acid and crotonic acid; and α-β-unsaturated dicarboxylic acids such as itaconic acid, fumaric acid, maleic acid and aconitic acid. Salts, esters or anhydrides of the α-β-unsaturated mono- or dicarboxylic acids described above may also be used. Examples include half esters of α-β-unsaturated dicarboxylic acids with C₁₋₄ alkanols, such as monomethyl fumarate; cyclic anhydrides of α-β-unsaturated dicarboxylic acids such as maleic anhydride, itaconic anhydride and citraconic anhydride; and esters of acrylic acid or methacrylic acid with C₁₋₃₀ alkanols, such as ethyl acrylate, butyl acrylate, 2-ethylhexyl acrylate, dodecyl acrylate, hexadecyl acrylate, and octadecyl acrylate.

Optionally, other ethylenically unsaturated monomers can be copolymerized into the carboxylic acid polymer backbone. Example of such other ethylenically unsaturated monomers include styrene, vinyl acetate, ethylene, butadiene, acrylonitrile and mixtures thereof. Carboxylic acid polymers may preferably have a molecular weight of at least 1 million Daltons.

Suitable examples include crosslinked copolymers polymerized from C₁₋₄ alkyl acrylate or methacrylate (e.g. ethyl acrylate) with one or more comonomers selected from acrylic acid, methacrylic acid and mixtures thereof. Such materials may generally be referred to under the INCI name of Acrylates Copolymer. Commercially available examples include Aculyn^{®} 33 from Rohm and Haas.

Also suitable are crosslinked copolymers polymerized from C₁₀₋₃₀ alkyl esters of acrylic or methacrylic acid with one or more comonomers selected from acrylic acid, methacrylic acid and their respective C₁₋₄ alkyl esters. Such materials may generally be referred to under the INCI name of Acrylates/C10-30 Alkyl Acrylate Crosspolymer. Commercially available examples include Carbopol^{®} polymers 1342 and 1382 from Lubrizol Advanced Materials.

Also suitable are optionally crosslinked copolymers of acrylic acid or methacrylic acid with alkyl acrylates and ethoxylated hydrophobically modified alkyl acrylates. Such materials may generally be referred to under the INCI names of Acrylates/Steareth-20 Methacrylate Copolymer, Acrylates/Beheneth-25 Methacrylate Copolymer, Acrylates/Steareth-20 Methacrylate Crosspolymer and Acrylates/Palmeth-25 Acrylates Copolymer. Commercially available examples include Aculyn^{®} 22, 28 or 88 from Rohm & Haas and Synthalen^{®} from 3V Sigma.

It is preferred that the carboxylic acid is a Carbomer, such as homopolymers of acrylic acid crosslinked with an allyl ether of pentaerythritol or an allyl ether of sucrose.

Mixtures of any of the aforementioned materials may also be used.

Preferably the hair care composition of the invention comprises 0.1 to 3.0%, more preferably 0.4 to 1.5% carboxylic acid polymer by weight of the composition.

In formulations containing anionic polymeric rheology modifiers such as the carboxylic acid polymers described above, it is often necessary to neutralize at least a portion of the free carboxyl groups by the addition of an inorganic or organic base. Examples of suitable inorganic or organic bases include alkali metal hydroxides (e.g. sodium or potassium hydroxide), sodium carbonate, ammonium hydroxide, methylamine, diethylamine, trimethylamine, monoethanolamine, triethanolamine and mixtures thereof.

The hair care composition of the invention may also comprise a nonionic polymeric rheology modifier which is selected from one or more nonionic cellulose ethers.

Suitable nonionic cellulose ethers or use as the nonionic polymeric rheology modifier in the invention include (C₁₋₃ alkyl) cellulose ethers, such as methyl cellulose and ethyl cellulose; hydroxy (C₁₋₃ alkyl) cellulose ethers, such as hydroxyethyl cellulose and hydroxypropyl cellulose; mixed hydroxy (C₁₋₃ alkyl) cellulose ethers, such as hydroxyethyl hydroxypropyl cellulose; and (C₁₋₃ alkyl) hydroxy (C₁₋₃ alkyl) cellulose ethers, such as hydroxyethyl methylcellulose and hydroxypropyl methylcellulose.

Preferred nonionic cellulose ethers for use as the nonionic polymeric rheology modifier in the invention are water-soluble nonionic cellulose ethers such as methylcellulose and hydroxypropyl methylcellulose. The term "water-soluble" in this context denotes a solubility in water of at least 1 gram, more preferably at least 3 grams, most preferably at least 5 grams in 100 grams of distilled water at 25° C. and 1 atmosphere. This level indicates production of a macroscopically isotropic or transparent, coloured or colourless solution.

Methyl cellulose and hydroxypropyl methylcellulose are commercially available in a number of viscosity grades from Dow Chemical as their METHOCEL^{®} trademark series.

Mixtures of any nonionic cellulose ethers may also be suitable. In a typical composition according to the invention the level of nonionic cellulose ethers will generally range from about 0.01 to about 2.0%, and preferably ranges from 0.1 to 0.5%, more preferably from 0.1 to 0.3%, by weight based on the total weight of the composition.

Preferably the haircare composition of the invention comprises 0.1 to 0.3% by weight nonionic cellulose ether.

It is further preferred that the hair care composition of the invention comprises a cosmetic ingredient. Preferably the cosmetic ingredient is selected from the group consisting of a silicone, an antibacterial agent other than antidandruff agents, a foam booster, a perfume, encapsulates (for example encapsulated fragrance) a dye, a colouring agent, a pigment, a preservative, a thickener, a protein, a phosphate ester, a buffering agent, a pH adjusting agent, a pearlescer (for example; mica, titanium dioxide, titanium dioxide coated mica, ethylene glycol distearate (INCI glycol distearate)) and/or opacifier, a viscosity modifier, an emollient, a sunscreen, an emulsifier, a sensate active (for example menthol and menthol derivatives), vitamins, mineral oils, essential oils, lipids, natural actives, glycerin, natural hair nutrients such as botanical extracts, fruit extracts, sugar derivatives and amino acids, microcrystalline cellulose and mixtures thereof.

Preferably, the hair care composition of the present invention includes from 0.01 to 20 wt% of the at least one cosmetic ingredient, more preferably from 0.05 to 10 wt%, still more preferably from 0.075 to 7.5 wt% and most preferably, from 0.1 to 5 wt% of the at least one cosmetic ingredient, by weight of the total composition.

The hair care composition of the present invention may also comprise synergistic antimicrobial compounds that give synergistic antimicrobial benefit when used in combination with the piroctone olamine to enhance its properties and further inhibit the growth of Malassezia furfur. Non-limiting examples of these compounds include compounds having alcoholic groups (e.g. honokiol, magnolol or paeonol), Piperazines and a phenolic compound found in natural plant extract viz. thymol and terpeniol.

The composition may additionally comprise a vitamin B3 compound. The preferred vitamin B3 compound is niacinamide.

Niacinamide is known for secretion of AMPs (Anti-Microbial Proteins) from keratinocytes. The AMPs thus secreted provides for improving immunity of e.g. the scalp. Thus with the use of niacinamide, the anti-dandruff efficacy can be enhanced not just through anti-fungal activity but by boosting the scalp's own protection shield against germs, through use of niacinamide. This combination could provide further long-lasting protection e.g. up to 24 hours of protection against germs.

When present, it is preferred that the hair care composition of the invention comprises 0.1 to 5% niacinamide, more preferably 0.5 to 5%, furthermore preferably 0.5 to 3%, and optimally 1.0 to 3.0% by weight of the composition.

### Method and Use

The present invention also provides for a method of treating dandruff comprising a step of topical applying the composition of the first aspect on to a desired scalp surface. The method is non-therapeutic in nature. Preferably it is for cosmetic purpose.

The present invention also provides for use of a composition of the first aspect to treat dandruff on desired scalp surface. The use is non-therapeutic in nature, preferably cosmetic in nature.

### Mode of Use

The hair care composition of the invention is primarily intended for topical application to hair and scalp.

When the composition is a shampoo, it is topically applied to the hair and then massaged into the hair and scalp. Then it is rinsed with water prior to drying the hair.

The invention will be further illustrated by the following, non-limiting Examples, in which all percentages quoted are by weight based on total weight unless otherwise stated.

The examples are intended to illustrate the invention and are not intended to limit the invention to those examples *per se.*

### Examples

### Example 1: Stability of Octopirox^{®} of the shampoo compositions with the presence of metal salts, and the impact of the weight ratio of polyaspartate or its derivative to Octopirox^{®}.

### Preparation of hair care compositions

The following shampoo compositions as shown in Table 1 were prepared:

**Table 1**

| **Ingredients (wt%)** | **Compositions** | | | |
|---|---|---|---|---|
| | **A** | **B** | **1** | **2** |
| SLES | 1.3 | 1.3 | 1.3 | 1.3 |
| CAPB | 0.15 | 0.15 | 0.15 | 0.15 |
| Octopirox^{®} | 0.1 | 0.1 | 0.1 | 0.1 |
| Sodium polyaspartate | - | 0.05 | 0.1 | 0.2 |
| Water | To 100 | To 100 | To 100 | To 100 |

### Stability of Octopirox^{®} test method

0.1% FeCl3·6H2O solution were added to above compositions to mimic the presence of iron salt from tap water during and after applying the products and sweat. Then the aqueous solutions diluted 20 times with menthol in 2 mL ultra performance liquid chromatography (UPLC) brown vial for measurement.

A Waters^{®} ACQUITY ultra performance liquid chromatography (UPLC, Waters, Manchester, UK) was used for the sample analysis. MassLynx software (version 4.1) was used for instrument control and data acquisition. Separation was carried out on a Waters Acquity UPLC BEH C18 column (2.1 × 100 mm, 1.7 µm particle size). The mobile phase A was composed of 0.1% formic acid + 5µM medronic acid in DI water and the mobile phase B was composed of 0.1% formic acid+ 5µM medronic acid in acetonitrile, programmed in a gradient mode. The sample was measured by photo-diode array (PDA) detector at absorption wavelength of 302 nm.

The values in Table 2 below are the free Octopirox^{®} concentration in the compositions:

**Table 2**

| **Compositions** | **A** | **B** | **1** | **2** |
|---|---|---|---|---|
| Free Octopirox^{®} (ppm) | 41.73±2.01 | 70.83±0.36 | 111.23±3.77 | 207.70±8.43 |

The results showed that composition 1 and 2 of the present invention provided significantly better stability of piroctone olamine than compositions A (without polyaspartate or its derivative) and B (weight ratio of polyaspartate or its derivative to piroctone olamine lower than 2:3) with the presence of metal salts.

### Example 2: Impact of surfactant system on the stability of Octopirox^{®} with the presence of metal salts.

The following compositions as shown in Table 3 were prepared.

**Table 3**

| **Ingredients (wt%)** | **Compositions** | | |
|---|---|---|---|
| | **C** | **D** | **3** |
| SLES | 1.3 | 1.3 | 1.3 |
| CAPB | 0.15 | - | 0.15 |
| CMEA | - | 0.15 | - |
| Carbomer | 0.035 | 0.035 | 0.035 |
| Octopirox^{®} | 0.05 | 0.05 | 0.05 |
| Sodium polyaspartate | 0 | 0.1 | 0.1 |
| Water | To 100 | To 100 | To 100 |

### Stability of Octopirox^{®} with the presence of metal salt

10% FeCl₃·6H₂O aqueous solution was prepared and added into the above shampoo samples. The final FeCl₃·6H₂O concentration was 0.1% and the percentages of other actives were not affected. The above shampoo compositions were colorless, however they turned red immediately when iron salt was added. It was found by the inventor when FeCl3·6H2O aqueous solution was added to shampoo composition without Octopirox^{®} it turned light yellow. Therefore, it was presumed that the red color came from the complex formed by Octopirox^{®} and iron ions.

The colour of above compositions after adding FeCl₃·6H₂O was measured with Hunterlab UltraScan Pro (Hunter Associates Laboratory, Inc). EasyMatchQC Version 4.12 software was used for instrument control and data acquisition. The measurement mode is TTRAN-Total Transmission, and the color of DI water was set as background.

The values in Table 4 below indicate the intensity of redness. Higher intensity of redness indicates more Octopirox^{®} reacted with metal salts and less free Octopirox^{®} left in the composition.

**Table 4**

| **Compositions** | **C** | **D** | **3** |
|---|---|---|---|
| The intensity of redness (a.u.) | 46.95±0.026 | 37.32±0.016 | 28.42±0.008 |

The data in Table 4 clearly indicates that the stability of Octopirox^{®} with the presence of metal salts is improved when the composition in accordance with this invention is used (Composition 3) during the experiments, as compared to corresponding examples outside the invention (Composition C - not containing polyaspartate or its derivative, Composition D - not using combination of surfactants of the present invention).

### Example 3: Impact on the stability of Octopirox^{®} with the presence of metal ions from polyaspartate or its derivative comparing with other chelating agents which is a polymer or its derivative.

The following compositions as shown in Table-5 were prepared.

**Table 5**

| **Ingredients (wt%)** | **Compositions** | | | | | |
|---|---|---|---|---|---|---|
| | **E** | **F** | **G** | **H** | **I** | **4** |
| Octopirox^{®} | 0.003 | 0.003 | 0.003 | 0.003 | 0.003 | 0.003 |
| Polyacrylic acid | | 0.25 | | | | |
| PVM/MA copolymer (Gantrez S97) | | | 0.25 | | | |
| polyglutamic acid | | | | 0.25 | | |
| Poly-arginine | | | | | 0.25 | |
| Sodium polyaspartate | | | | | | 0.25 |
| Water: Ethanol=1:1 | To 100 | To 100 | To 100 | To 100 | To 100 | To 100 |

### Stability of Octopirox^{®} test method

0.01% FeCl3·6H2O solution were added to above compositions to mimic the presence of iron salt from sweat. Then the aqueous solutions diluted 20 times with menthol in 2 mL ultra-performance liquid chromatography (UPLC) brown vial for measurement.

The values in Table 6 below are the free Octopirox^{®} percentage (the original free Octopirox^{®} is considered as 100%):

**Table 6**

| **Compositions** | **E** | **F** | **G** | **H** | **I** | **4** |
|---|---|---|---|---|---|---|
| Free Octopirox^{®} (%) | 64.99± 0.32 | 80.55± 0.40 | 79.70± 0.04 | 65.51± 8.43 | 73.48± 0.52 | 99.30± 0.50 |

The results showed that composition 4 of the present invention provided significantly better stability of piroctone olamine than compositions E-I (without polyaspartate or its derivative) with the presence of metal salts.

## Claims

1. A hair care composition comprising:
(i) an anionic surfactant;
(ii) an amphoteric or zwitterionic surfactant;
(iii) piroctone olamine;
(iv) a polyaspartate or its derivative presented by the structure of Formula 1 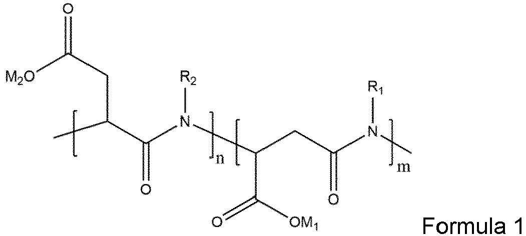 where the sum of m+n is from 2 to 10000, R1, R2 are independently selected from the group consisting of H, OH, (O)ₘG, m is 0 or 1, G is independently selected from the group consisting of H and a substituted or unsubstituted C1-C12 organic group, M1, M2 are independently selected from the group consisting of H, metal salt or ammonium salt, wherein non-limiting examples of the metal salts include Na, K, Ca, Mg, Al salts; and
(v) a cosmetically acceptable carrier.
wherein the weight ratio of said polyaspartate or its derivative to said piroctone olamine in the composition is at least 2:3.

2. A hair care composition as claimed in claim 1 wherein said polyaspartate or its derivative represented by the structure of formula 1 wherein sum of m+n is from 10 to 50, R1, R2 are H, M1, M2 are independently selected from the group consisting of H, metal salt or ammonium salt, wherein non-limiting examples of the metal salts include Na, K, Ca, Mg, Al salts.

3. A hair care composition as claimed in claim 1 or 2 wherein said polyaspartate derivative is sodium polyaspartate.

4. A hair care composition as claimed in any of claims 1 to 3 wherein said anionic surfactant is selected from alkyl sulfate surfactant, alkyl ether sulfate surfactant, or mixture thereof.

5. A hair care composition as claimed in claim 4 wherein said anionic surfactant is an alkyl ether sulfate surfactant which is sodium lauryl ether sulfate.

6. A hair care composition as claimed in any of claims 1 to 5 wherein said amphoteric surfactant is a betaine surfactant.

7. A hair care composition as claimed in claim 6 wherein said betaine surfactant is one of alkyl amidopropyl betaines.

8. A hair care composition as claimed in claim 6 or 7 wherein said betaine surfactant is cocamidopropyl betaine.

9. A hair care composition as claimed in any of claims 1 to 8 wherein amount of said polyaspartate or its derivative is 0.1 to 10 wt%.

10. A hair care composition as claimed in any of claims 1 to 9 wherein amount of said piroctone olamine is 0.1 to 10 wt%.

11. A hair care composition as claimed in any of claims 1 to 10 wherein amount of said anionic surfactant is 0.1 to 20 wt%.

12. A hair care composition as claimed in any of claims 1 to 11 wherein amount of said amphoteric surfactant is 0.1 to 10 wt%.

13. A hair care composition as claimed in any of claims 1 to 12 wherein said composition comprises a cationic polymer.

14. A hair care composition as claimed in any of claims 1 to 13 wherein the composition is a shampoo.

15. The composition as defined in any of claims 1 to 14 for use in a method of treating dandruff by topical application onto a desired scalp surface

## Patentansprüche

1. Haarpflegezusammensetzung, umfassend:
(i) ein anionisches Tensid;
(ii) ein amphoteres oder zwitterionisches Tensid;
(iii) Piroctonolamin;
(iv) ein Polyaspartat oder sein Derivat, durch die Struktur der Formel 1 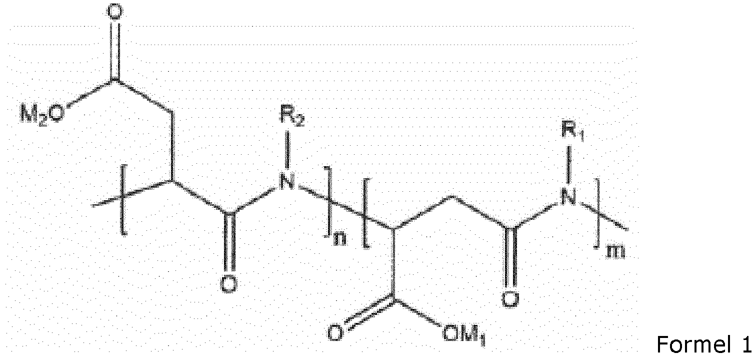 dargestellt,
wobei die Summe von m+n 2 bis 10000 ist,
R1, R2 unabhängig voneinander aus der Gruppe ausgewählt sind, bestehend aus H, OH, (O)ₘG,
m 0 oder 1 ist,
G unabhängig voneinander aus der Gruppe ausgewählt ist, bestehend aus
H und einer substituierten oder unsubstituierten organischen C1-C12-Gruppe,
M1, M2 unabhängig voneinander aus der Gruppe ausgewählt sind, bestehend aus H, Metallsalz oder Ammoniumsalz, wobei die nicht beschränkenden Beispiele der Metallsalze Na-, K-, Ca-, Mg-, Al-Salze umfassen; und
(v) einen kosmetisch akzeptablen Träger,
wobei das Gewichtsverhältnis des Polyaspartats oder seines Derivats zum Piroctonolamin in der Zusammensetzung mindestens 2:3 beträgt.

2. Haarpflegezusammensetzung, wie im Anspruch 1 beansprucht, wobei das Polyaspartat oder sein Derivat durch die Struktur der Formel 1 dargestellt sind,
wobei die Summe von m+n 10 bis 50 beträgt,
R1, R2 H sind,
M1, M2 unabhängig voneinander aus der Gruppe ausgewählt sind, bestehend aus H, Metallsalz oder Ammoniumsalz, wobei nicht beschränkende Beispiele der Metallsalze Na-, K-, Ca-, Mg-, Al-Salze umfassen.

3. Haarpflegezusammensetzung, wie in Anspruch 1 oder 2 beansprucht, wobei das Polyaspartatderivat Natriumpolyaspartat ist.

4. Haarpflegezusammensetzung, wie in einem der Ansprüche 1 bis 3 beansprucht, wobei das anionische Tensid unter Alkylsulfat-Tensid, Alkylethersulfat-Tensid oder einer Mischung davon ausgewählt ist.

5. Haarpflegezusammensetzung, wie im Anspruch 4 beansprucht, wobei das anionische Tensid ein Alkylethersulfat-Tensid ist, das Natriumlaurylethersulfat ist.

6. Haarpflegezusammensetzung, wie in einem der Ansprüche 1 bis 5 beansprucht, wobei das amphotere Tensid Betain-Tensid ist.

7. Haarpflegezusammensetzung, wie im Anspruch 6 beansprucht, wobei das Betain-Tensid eines von Alkylamidopropylbetainen ist.

8. Haarpflegezusammensetzung, wie im Anspruch 6 oder 7 beansprucht, wobei das Betain-Tensid Cocamidopropylbetain ist.

9. Haarpflegezusammensetzung, wie in einem der Ansprüche 1 bis 8 beansprucht, wobei die Menge des Polyaspartats oder seines Derivats 0,1 bis 10 Gew.-% beträgt.

10. Haarpflegezusammensetzung, wie in einem der Ansprüche 1 bis 9 beansprucht, wobei die Menge des Piroctonolamins 0,1 bis 10 Gew.-% beträgt.

11. Haarpflegezusammensetzung, wie in einem der Ansprüche 1 bis 10 beansprucht, wobei die Menge des anionischen Tensids 0,1 bis 20 Gew.-% beträgt.

12. Haarpflegezusammensetzung, wie in einem der Ansprüche 1 bis 11 beansprucht, wobei die Menge des amphoteren Tensids 0,1 bis 10 Gew.-% beträgt.

13. Haarpflegezusammensetzung, wie in einem der Ansprüche 1 bis 12 beansprucht, wobei die Zusammensetzung ein kationisches Polymer umfasst.

14. Haarpflegezusammensetzung, wie in einem der Ansprüche 1 bis 13 beansprucht, wobei die Zusammensetzung ein Shampoo ist.

15. Zusammensetzung, wie in einem der Ansprüche 1 bis 14 definiert, zur Verwendung in einem Verfahren zur Behandlung von Schuppen durch topischen Auftrag auf die Kopfhautoberfläche.

## Revendications

1. Composition de soin capillaire comprenant :
(i) un tensioactif anionique ;
(ii) un tensioactif amphotère ou zwittérionique ;
(iii) de la piroctone olamine ;
(iv) un polyaspartate ou son dérivé représenté par la structure de formule 1 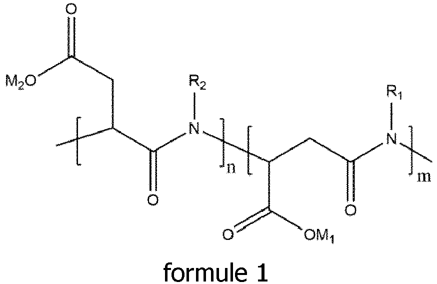 dans laquelle la somme m+n vaut de 2 à 10 000, R1 et R2 sont indépendamment choisis dans l'ensemble constitué par H, OH, (O)ₘG, m vaut 0 ou 1, G est indépendamment choisi dans l'ensemble constitué par H et un groupe organique en C1 à C12 substitué ou non substitué, M1 et M2 sont indépendamment choisis dans l'ensemble constitué par H, un sel métallique et un sel d'ammonium, dans laquelle des exemples non limitatifs des sels métalliques comprennent les sels de Na, K, Ca, Mg, Al ; et
(v) un véhicule acceptable du point de vue cosmétique,
dans laquelle le rapport en poids dudit polyaspartate ou de son dérivé à ladite piroctone olamine dans la composition est d'au moins 2/3.

2. Composition de soin capillaire selon la revendication 1, dans laquelle ledit polyaspartate ou son dérivé est représenté par la structure de formule 1 dans laquelle la somme m+n vaut de 10 à 50, R1 et R2 sont H, M1 et M2 sont indépendamment choisis dans l'ensemble constitué par H, un sel métallique et un sel d'ammonium, dans laquelle des exemples non limitatifs des sels métalliques comprennent les sels de Na, K, Ca, Mg, Al.

3. Composition de soin capillaire selon la revendication 1 ou 2, dans laquelle ledit dérivé de polyaspartate est le polyaspartate de sodium.

4. Composition de soin capillaire selon l'une quelconque des revendications 1 à 3, dans laquelle ledit tensioactif anionique est choisi parmi un tensioactif alkylsulfate, un tensioactif alkyl-éthersulfate, et leurs mélanges.

5. Composition de soin capillaire selon la revendication 4, dans laquelle ledit tensioactif anionique est un tensioactif alkyl-éthersulfate qui est le lauryl-éthersulfate de sodium.

6. Composition de soin capillaire selon l'une quelconque des revendications 1 à 5, dans laquelle ledit tensioactif amphotère est un tensioactif de type bétaïne.

7. Composition de soin capillaire selon la revendication 6, dans laquelle ledit tensioactif de type bétaïne est l'une des alkyl-amidopropyl-bétaïnes.

8. Composition de soin capillaire selon la revendication 6 ou 7, dans laquelle ledit tensioactif de type bétaïne est le (coprah-yl)amidopropyl-bétaïne.

9. Composition de soin capillaire selon l'une quelconque des revendications 1 à 8, dans laquelle la quantité dudit polyaspartate ou de son dérivé est de 0,1 à 10 % en poids.

10. Composition de soin capillaire selon l'une quelconque des revendications 1 à 9, dans laquelle la quantité de ladite piroctone olamine est de 0,1 à 10 % en poids.

11. Composition de soin capillaire selon l'une quelconque des revendications 1 à 10, dans laquelle la quantité dudit tensioactif anionique est de 0,1 à 20 % en poids.

12. Composition de soin capillaire selon l'une quelconque des revendications 1 à 11, dans laquelle la quantité dudit tensioactif amphotère est de 0,1 à 10 % en poids.

13. Composition de soin capillaire selon l'une quelconque des revendications 1 à 12, laquelle composition comprend un polymère cationique.

14. Composition de soin capillaire selon l'une quelconque des revendications 1 à 13, laquelle composition est un shampooing.

15. Composition de soin capillaire selon l'une quelconque des revendications 1 à 14, pour une utilisation dans une méthode de traitement des pellicules par application topique sur une surface souhaitée du cuir chevelu.
